Europäisches Patentamt

European Patent Office

Office européen des brevets

(11) Publication number: 0 287 883
A1

# EUROPEAN PATENT APPLICATION

(21) Application number: 88105365.6

(22) Date of filing: 02.04.88

(51) Int. Cl.⁴: **B01L 3/00 , G01N 33/52**

(30) Priority: 13.04.87 US 37876

(43) Date of publication of application:
26.10.88 Bulletin 88/43

(84) Designated Contracting States:
DE FR GB

(71) Applicant: **MILES INC.**
**1127 Myrtle Street**
**Elkhart Indiana 46514(US)**

(72) Inventor: **Blatt, Joel M.**
**17100 Barryknoll Way**
**Granger Indiana 46530(US)**

(74) Representative: **Adrian, Albert, Dr. et al**
**c/o BAYER AG Konzernverwaltung RP**
**Patentabteilung**
**D-5090 Leverkusen 1, Bayerwerk(DE)**

(54) Test strip device with volume metering capillary gap.

(57) A method for quick and rapid reading of a dip and read device (1) in a liquid sample using a device (1) having a reactive surface (4) and a capillary gap for controlled fluid flow comprising two opposing surface areas (2, 4) spaced apart throughout a capillary zone (5) of intended liquid transport at a distance no greater than that which will maintain a capillary flow of liquid introduced therebetween, means (5) to permit introduction of a test specimen between these surfaces, there being formed between said opposing surfaces an air vent means (26, 37) to permit any entrapped air to quickly escape from the sample chamber and thereby avoid interference with the observation of the test results.

FIG. I

EP 0 287 883 A1

## TEST STRIP DEVICE WITH VOLUME METERING CAPILLARY GAP

### Field of the Invention

The present invention relates to a test strip device and method for testing a determined amount of a liquid sample and, more particularly, to a test strip device and method that permits the device to be immersed in relatively large volumes of liquids and the control of a determined amount of said liquid for contact onto a reactive surface which enables visual or other sensing means to ascertain the presence of a sought after component in the liquid sample and/or the amount of said component.

### Description of the Prior Art

A considerable variety of testing and analytical elements have been developed over the years. Chemical analysis of liquids such as water and foodstuffs, and especially biological fluids such as blood and urine is often desirable or necessary for the health and welfare of any population. Many different types of testing devices to facilitate such analyses have been developed in the industry. Some of these devices for liquid analysis require the addition of another liquid reagent which is capable of undergoing a reaction leading to the formation of a colored material or other detectable change. Other systems depend on a dry system such as pH papers and the like, where the paper or other highly absorbent carrier is impregnated with a material which is chemically reactive or responsive in contact with the liquid containing the substance under analysis, termed an "analyte", and generates a color or other type of change. Depending upon the selection of responsive material, the change is usually qualitative or at best semi-quantitative. For diagnostic chemical analysis wherein the testing of biological fluids such as blood, plasma, urine and the like are utilized, it is preferable to produce highly quantitative results rapidly and conveniently. Also, it is desirable to precisely control and monitor the amount of liquid specimen that is subjected to the test. This is important especially in tests which involve machine reading of test substrates where it is necessary that a calibrated amount of the test specimen is exposed to the test substrate so that the proper reaction will take place and that any interference with optical detection or other detection of color changes is avoided.

A variety of devices and methods have been developed for transporting liquid in a controlled and predetermined flow pattern. Many of such items have been concerned with uncontrolled and undirected capillary flow of the liquid across surfaces. Some problems that have been encountered with uncontrolled flow include formation of trapped air pockets and incomplete wetting of certain portions of the surface. Air pockets create problems when the test device is examined through a microscope or other automatic methods because the examination of the liquid and/or the wetted surfaces results in different test data being collected. The examinations and automated systems are based on a presumption of the presence of the liquid in the scanning area and therefore the absence of the liquid in the relevant scanning area will throw off the value of the reading and will give an unreliable result. The problem of air pockets is a common occurrence particularly when dealing with configurations which have sharp corners and synthetic resin surfaces which are generally hydrophobic.

A variety of different types of liquid transport devices have been developed in the prior art including that shown in Columbus, U.S. 4,233,029, which describes a device containing a means for directing capillary flow along predetermined paths by use of grooves in the opposed surfaces of a capillary chamber.

Another configuration for the transport of a liquid test specimen is shown in Columbus, U.S. 4,254,083, which provides for an exterior drop receiving surface containing a particular opening configuration which is intended to facilitate the centering of the drop.

Buissiere et al., U.S. 3,690,836, describe a device consisting of a capillary space between two plastic sheets which are sealed in a continous perimeter and which enclose an uncompressed absorbent material which fills the capillary space. At least one opening at the top sheet provides for access to the reaction chamber.

A liquid transport device which provides for diversion of capillary flow into a second zone is shown in Columbus, U.S. 4,473,457. The device has two pathways for flow of the specimen and permits the introduction of two different specimens through two apertures. The two liquids then will flow towards and into a common area. The configuration of the structure of Columbus permits potentiometric determinations to be made. See also Columbus, U.S. 4,302,313, which shows a device suitable for potentionmetric analysis of liquid ions. Special grooved surfaces under the member 36 are said to control capillary flow.

Another device is shown by Columbus, U.S.

4,271,119, which has a downstream diverting aperture in a wall member of a first capillary zone which provides capillary flow into a second capillary zone extending from that wall member.

Columbus, U.S. 4,323,536, discloses a multi-analyte test device. Liquid flow control means are included such that liquid is confined to a plurality of flow paths.

In the U.K. published application G.B. 2090659A, there is shown an analytical device with a self-filling metering channel for use by touching the metering channel inlet to a drop of blood.

## Summary of the Invention

The present invention pertains to a test strip device capable of volume metering of liquid samples onto a reactive surface in a capillary gap device that can be used for the testing of large volumes of liquid specimens by the so-called "dip and read" method while avoiding air entrapment problems associated with prior know devices. The device provides for a rapid and uniform distribution of a predetermined volume of a liquid test specimen onto a reactive surface for the determination of a particular component or components that may or may not be present in the liquid test specimen. The volume of sample applied to the surface is limited to that amount which resides within a sample capillary gap or sample reading chamber. Excess sample simply runs off the device when the device is removed after dipping into the test specimen liquid. The configuration and structure of the device is such that entrapment of air is greatly reduced which thereby makes the devices of the present invention especially well suited for use in dip and read applications when there is a need to test large volumes of liquid, such as is the case when performing urine analysis.

Application of a uniformly distributed sample onto a reactive surface and control of the sample volume are important features of the present invention and thereby enable avoidance of problems associated with dry reagent films and papers.

The aforementioned advantages permit one to choose a sample volume appropriate to the chemistry and reactivity of the reactive material by constructing the device in dimensions so that the length, breadth and depth of the capillary gap and hence the total volume entrained by the sample reading chamber of the device can be tailored for specific end uses if desired.

With the devices of the present invention, no special measures need be taken to remove excess fluid beyond the capacity of the capillary gap. Any excess sample will simply drain off the outer surfaces of the device.

Other features and advantages of the present invention will become apparent from the following detailed description taken in conjunction with the drawings.

## Brief Description of the Drawings

Figure 1 is a perspective view of one embodiment of the capillary gap device of the present invention;

Figure 2 is a perspective view of another embodiment of the present invention; and

Figure 3 is a top view of an alternative arrangement of the present invention, which is partially assembled.

Major problems associated with prior art devices are solved by the device of the invention. For example, with dry reagent films and papers, it is difficult utilizing prior art techniques to obtain an application of an uniformly distributed sample over a finite surface area of a test surface. In many instances, the sample will not travel into the sample chamber under proper conditions; too much sample is in contact with the sample chamber or not enough of the liquid is in contact therewith. The device of the present invention permits close and carefully monitored control of the sample volume so that only a previously determined calibrated amount of liquid to be analyzed will be in contact with the test surface. Therefore, these advantages permit one to choose a sample volume that is appropriate to the particular test that is being carried out taking into consideration the nature of the fluid that is being tested and the nature of the reagent film.

The device of the invention presents a novel construction and configuration whereby prior art problems associated with air entrapment are essentially eliminated. Thus, the dimensions and arrangement of elements are such that any air can pass out of the device and be released in an expeditious manner to avoid interference with the test readings.

The device of the present invention can be fabricated in various different sizes. Accordingly, the dimension of the capillary gap can be varied as desired. Capillary gap devices can be made in various sizes depending upon the total volume of the sample that is desired to be entrained. This will depend at least in part upon the particular means chosen for reading the results; i.e., either automatic or visual means. Excess sample simply drains off and therefore does not interfere with obtaining a proper reading.

It is important to note that the present invention is not simply a fluid transport or spreading device but instead is a volume metering device which is designed to accommodate a range of sample volumes from a minimum of about 5 to 10 micro liters up to about 100 to 200 micro liters without washing or wiping off the excess liquid and without associated air entrapment problems.

It is therefore an important feature of the present invention to provide a fluid metering test strip device with a capillary gap structure containing a sample chamber of a defined volume.

It is a further feature of the present invention to provide a capillary gap device which permits rapid air release and prevents interference with the ability to read test results by manual or machine methods.

A further feature of the present invention is to provide a test strip device that is open on at least two sides, preferably four sides, and thereby permits rapid filling with sample liquid and quick release of any entrapped air.

A further feature of the present invention is to provide a test strip device for immersion into relatively large liquid specimens and having an air vent channel with an opening that is always intended to be positioned or held above the liquid into which the test device is immersed.

A still further feature of the present invention is to provide a capillary gap device having a multiple read area which avoids interference due to air entrapped by providing an air vent channel in the device for rapid release of air.

Detailed Description of Invention

Described in further detail, the device of this invention features a capillary transport for biological liquids particularly whole blood which can be visually inspected or subjected to an automated system for sample reading. The device can be utilized by immersions into any liquid to be tested for the presence or absence of an analyte by the so-called "dip and read" system. The capillary gap controls the amount of the liquid carried through an opening port from the liquid specimen source to the reactive surface or test matrix. The device of the present invention features the metering of the fluid into a capillary gap containing a sample chamber of a defined volume.

The device of the present features appropriate air vent channel means so as to permit rapid and complete venting of any entrapped air without interfering with the reading and observation of the device.

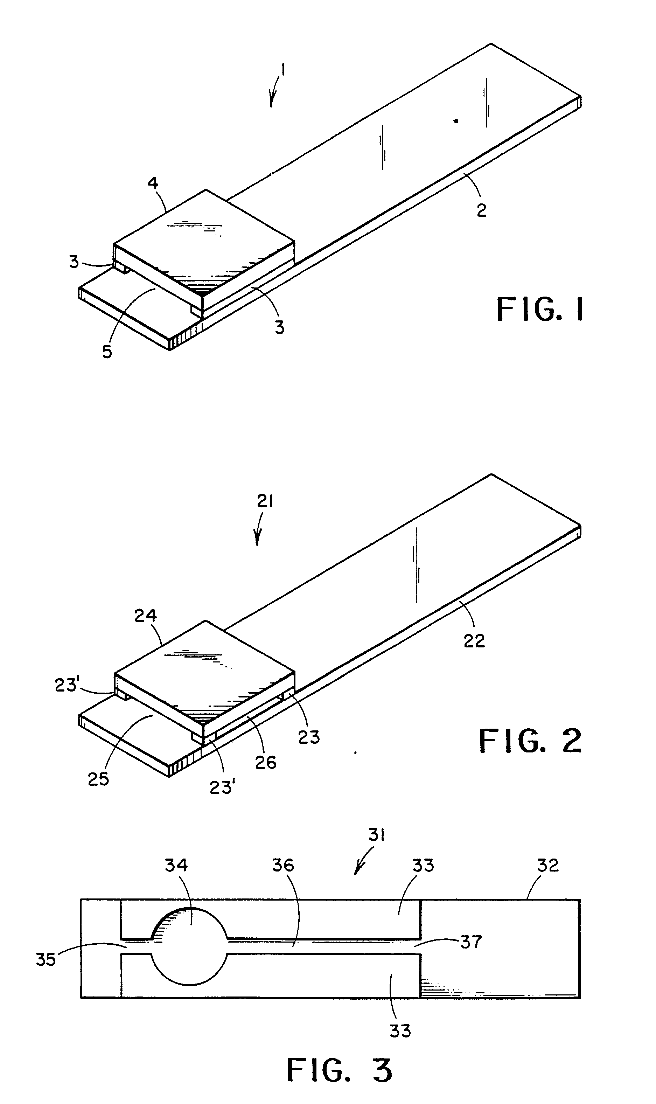

The device of the present invention as represented by the embodiment of the invention illustrated in Figure 1 includes a capillary device 1, of generally rectangular geometry, having a major axis and a minor axis and including a lower extended member 2 which serves as the strip handle, an intermediate spacer 3, and a top member 4. The top member or layer comprises the reagent detection means. The top is constructed as may be necessary or desirable to permit visual observation or recordation of the test result by manual or machine means.

The spacer layer 3 defines the internal capillary gap or chamber and as shown in Figure 1 corresponds to two rectangular members of length equal to be fitted flush with the outside edges of lower extended member 2. The dimension of the interior capillary gap space are indicated by the letters "l" for length, "b" for breadth and "d" for depth. These dimensions can be varied as desired in the manufacture of these devices depending at least in part on the type of test for which they are intended. Generally, the distance between the top member 4 and lower member 2, indicated by "d" ranges from 0.007 to 0.08 cm.

The top layer 4 is the reagent containing layer and can be a reagent impregnated fibrous layer or a gelatin coated layer. Any one of a wide variety of reagent layers or matrices, including powders, can be used in accordance with the invention. Many conventional reagent systems are available and the specific choice of which reagent selected will depend upon the tests to be carried out.

The top layer 4 can comprise a mono or multilayer reagent material or a reagent matrix of any conventional type.

In the manufacture of the test device of the invention, the top layer 4 can be cut or stamped from a suitable material, such as a monolayer or multilayer reagent material. Whether it be in monolayer or multilayer form, the reagent top layer can also be molded or shaped to include depending side or corner portions so as to eliminate the need for separate spacer member means as will be described in further detail.

The spacer layer 3 can be formed of any suitable material such as a thermoplastic material which, upon heating, can be utilized to adhere the top layer 4 to the lower layer 2. Any suitable, dimensionally stable thermoplastic material can be used for this purpose such as polyamides, polyethylene, polypropylene, PVC, copolymers thereof and the like. Alternatively, a separate adhesive composition can be interposed between the layers in a sufficient amount to adhere all layers together in a secure and permanent fashion. Such adhesive substances are known in the art and any suitable one can be used provided it does not react with any test specimens.

It will be understood that any suitable way of joining the layers together can be used. Among other ways of assembling the various layers are welding the layers by ultrasonic means. In a yet further variation, a mechanical clamp can be used to maintain the layers together.

The dimensions of the capillary gap device can vary widely but it has been found that a particularly useful dimension is a ratio of about 3 to 1 overall length vs. width; that is, 2.5 to 8.5 cm in overall length by 0.5 to 2.4 cm in width. A particularly useful configuration is 8.5 cm in length by 0.5 cm in width. The thickness of the test device can also vary and generally is 0.05 to 0.25 cm. Typically, the three layers can include a 0.02 cm thick reagent top layer, a plastic and adhesive spacer layer, which can be approximately 0.02 cm thick, and a lower layer which can be of a suitable thickness; e.g. 0.02 cm thick.

Figure 2 is a perspective view of a different embodiment of a capillary gap device 21 of the invention composed of three layers; i.e., a top or reagent layer 24, a plurality (i.e. four) of spacer means 23, 23' and a strip handle layer 22. In this embodiment, the spacer means is in the form of four spacer means 23, 23', etc. located at each of the four corners of the top reagent film layer 24. In this variation, there is provided air vent voids 26 on the sides of the device to permit any air entering the capillary channel 25 to escape easily and quickly. Further, in this embodiment, the spacer means can be contoured in a curved shape on the interior which avoid sharp corners and thereby facilitate liquid and gas flow.

In the embodiments shown in Figures 1 and 2, the capillary gap or channel 5, 25 is of considerable breadth, b, which is at least 1/3 and preferably at least 1/2 as large, as the entire width of the layer 2, 22. The capillary channel 5 in Figure 1 passes through for the entire length, l, of the top layer 4. In the embodiment shown in Figure 2, the air vent voids 26 located at the side of the device are also at least 1/3 and preferably at least 1/2 as wide as the length of the top layer 24. These large openings enable the rapid release of entrapped air.

Figure 3 shows a top view of still a further embodiment of a partially assembled capillary gap device 31 of the invention including an elongated handle layer 32, a spacer layer 33, a sample chamber read area 34, entry port 35, air vent channel 36 and air vent 37. The air vent channel 36 extends from and connects the sample chamber read area 34 with the air vent 37. Sample entry port 35 connects with the sample chamber read area 34. The surfaces are smoothly contoured, as may be seen, so that sharp corners are generally avoided. The reagent top layer is not shown, but will normally extend over the entire distance of the spacer layer 33. In the embodiment represented by Figure 3, the length of the air vent channel 36 and the location of the air vent 37 are such that in using the device, it would be intended that air vent 37 would always be located above the liquid level during the immersion step. While Figure 3 shows a single sample chamber read area 34, it is to be understood that the device 31 can be made so as to include a plurality of such chambers connected to each other in series along the length of the strip to enable multiple readings; i.e., determinations of different analytes in the same sample or duplicate determinations of the same analyte present in the tested sample.

In Figure 3, the spacer layer 33 is formed of a conventional plastic material that is heat deformable and is punched out or molded to form the channel 36 and chamber read area 34. The top and bottom surfaces of layer 33 can be formed with dimples, pyramids or projections formed thereon to provide for good welding and bonding together when all the layers are united.

According to another embodiment of the present invention (not shown in the drawings), there is provided a capillary device of generally rectangular geometry which is formed of a top reagent layer and the bottom handle layer. In this embodiment of the invention, which generally can have the same shape and configuration as the embodiments of Figures 1 and 2, there is no separate spacer layer. The capillary channel and/or sample chamber, and and other openings, ports and channels can be directly formed in the top layer or in the handle layer. These openings, passageways and chambers can be formed in the top layer by drilling, cutting out, molding or any other means of formation that may be convenient. The top layer and the handle can be joined together by adhesive other suitable means, e.g., snap fit.

As is the case with other embodiments, the bottom layer can be clear and transparent, or translucent to facilitate observation. It can also be opaque. Any convenient and known plastic or other synthetic material can be used for this purpose such as polystyrene, polyolefins, polyamides, polyesters and the like. Further, as the case with other embodiments, the dimensions of the capillary gap device are generally similar but can vary as convenient or as desired.

The reagent layer, either as a mono-or multilayer reagent material or matrix, can be conveniently secured to the strip layer or spacer by any suitable adhesive material (not shown) which can be separately applied in a sufficient amount to adhere both layers together in a secure, permanent fashion. Alternatively, depending upon the composition of lower layer and reagent layer, they can be thermally fused together or fused by laser means or any other suitable means as will be apparent to those skilled in the art.

The adhesive pattern in accordance with the desired configuration can be applied, e.g., printed or screened onto the inner surface of the covering reagent layer or the lower layer or both layers in whatever thickness is required.

In operation, the dip and read device of the present invention is dipped into a sample of urine or other fluid containing the desired analyte. The stick can be rapidly dipped into the fluid and quickly withdrawn. Any excess liquid quickly drains from the device. The total time required is usually just a few seconds, typically about 2 seconds, and it is limited only by the rate at which the device can be immersed and withdrawn from the sample liquid.

As explained above, the main purpose of the device of the invention is to provide a quick and easy means to enable the testing of large volume sample fluids without requiring any washing or wiping of excess sample and without interference by entrapped air. In the device shown herein, any air is quickly vented to the surroundings because of the configuration of the capillary gap itself, the side vents and/or the air vent channel extending above the surface of the sample.

Any reagent substance can be used for purposes of the invention provided the reagent contains at least one material that is interactive or responsive in the presence of an analyte positive liquid present in the specimen to be tested. In various instances, the interactive material can be responsive to an analyte or a precursor or a reaction product of an analyte to effect the production of a change within the element by virtue of the reactive material. Thus, the reagent layer is permeable to at least one component present in the sample and is preferably of a substantially uniform permeability to those substances which are tested for in the test specimen. The term "permeable" as used herein indicates the ability of a substance or the layer to be penetrated effectively by a material carried in the test liquid. Uniform permeability of a layer refers to permeability such that when a homogeneous liquid is provided uniformly to a surface of the layer, identical measurements of the concentration of such liquid within the layer can be made through different regions of the surface of the layer permitting substantially the same results, within about 10%, to be obtained for each measurement. Because of the uniform permeability, undesirable concentration gradients can be avoided in the reagent layer. Such reagent layers are well known in the art and any suitable one can be used for purposes of the invention.

One or more surface active agents can be utilized to coat the interior of the capillary gap in the device so as to permit and facilitate liquid transport of the specimen into the sample chamber and/or the excess liquid overflow compartment. A broad variety of ionic and nonionic surface active agents can be used for this purpose. For example, the well known ionic surface active agents such as alkali metal and alkyl sulfates, wherein the alkyl group has more than 8 carbon atoms, such as sodium dodecyl sulfate, can be utilized. Nonionic surface active agents such as the many examples set forth in McCutcheon's "Detergents and Emulsifyers" 1974, North American Edition by the Allured Publishing Corporation can be used.

Analytical elements of the present invention can be adapted for use in carrying out a wide variety of chemical analyses, not only in the field of clinical chemistry but in chemical research and in chemical process control laboratories.

The invention is well suited for use in clinical testing of body fluids, such as blood, blood serum and urine, since in this work a large number of repetitive tests are frequently conducted and test results are often needed a very short time after the sample is taken. In the field of blood analysis, for example, a multilayer element can be adapted for use in carrying out quantitative analyses for many of the blood components which are routinely measured. Thus, for example, the element can be readily adapted for use in the analysis of such blood components as urea nitrogen, chloride, glucose and uric acid, as well as many other components by appropriate choice of test reagents or other interactive materials. In analyzing blood with an analytical element of this invention, the blood cells may first be separated from the serum, by such means as centrifuging, and the serum applied to the element. However, it is not necessary to make such separation, especially if reflective spectrophotometric analysis techniques are used to quantify or otherwise analyze the reaction product formed in the element as whole blood can be applied directly to the element and the blood cells filtered out through the action of a filtering layer. The presence of these cells on the element will not interfere with spectophotometric analysis if it is carried out by reflection techniques.

Reagent layers in the devices of the invention can be permeable or porous to samples obtained from a metering or spreading layer or to reaction products thereof. A multilayer reagent layer can include a metering or spreading layer. As used herein, the term "permeability" includes permeability arising from porosity, ability to swell or any other characteristic. Reagent layers can also include a matrix in which an interactive material is distributed, i.e., dissolved or dispersed. The choice of a matrix material is, of course, variable and dependent on the intended use of the element. Desirable matrix materials can include hydrophilic materials such a hydrophilic colloids, preferably in the form of a water-swellable gel. Useful hydrophilic materials include both naturally occurring substances like gelatin, gelatin derivatives, hydrophilic cellulose derivatives, polysaccharides such as dextran, gum arabic, agarose and the like, and also synthetic substances such as water-soluble polyvinyl compounds like polyvinyl alcohol and polyvinyl pyrrolidone, acrylamide polymers, etc. Organophilic materials such as cellulose esters and the like can also be useful, and the choice of materials in any instance will reflect the use for which a particular element is intended.

To enhance permeability of the reagent layer if not porous, it is often useful to use a matrix material that is swellable in the solvent or dispersion medium or liquid under analysis. The choice of a reagent layer matrix in any given instance may also depend in part on its optical or other properties that could affect radiometric detection. The reagent layer should be non-interfering with respect to any intended result of the detection procedure. Also, it may be necessary to select material that is compatible with the application of an adjacent layer, such as by coating means, during preparation of the element. As an example, where the formation of discrete layers is desired and the intended analysis will be of aqueous liquids, it may be appropriate to select an essentially water soluble matrix for the reagent layer and essentially organosoluble or organodispersible ingredients for an adjacent layer, such as a spreading layer. In such manner, mutual solvent action is minimized and a clearly delineated layer structure can be formed. In many cases, to facilitate the formation within the spreading layer, it may be desirable to have the reagent layer of lower permeability than is the spreading layer itself. Relative permeability can be determined by well-known techniques.

In various embodiments of the present elements, the interactive material in the reagent layer interacts with the analyte material to which the element is responsive. In other embodiments, the interactive material can interact with a precursor or a product of an analyte, as appropriate in view of the analysis mechanism of choice. The term "interactive" is meant herein to refer to chemical reactivity such as reactivity by addition, protonation, decomposition, etc., activity as in the formation of an enzyme-substrate complex, activity as is produced as a result of enzymatic action as well as any other form or composition of chemical or physical interaction able to produce or promote within the element, such as in the reagent layer, the formation of a radiometrically detectable change, i.e., one that is detectable by suitable measurement of light or other electromagnetic radiation.

The distribution of interactive material can be obtained by dissolving or dispersing it in the reagent matrix material. Although uniform distributions are often preferred, they may not be necessary if the interactive material is, for example, an enzyme. Reagents or other interactive materials soluble in the liquid under analysis can advantageously be immobilized in the reagent layer, particularly when the reagent layer is porous.

The particular interactive materials that can be distributed within a reagent layer will depend on the analysis of choice. In the case of glucose analysis, a ferricyanide compound can be used. Glucose reacts with ferricyanide and the reaction causes a decrease in the yellow color characteristic of ferricyanide. In testing for uric acid, as in blood of serum, a mixture of copper sulfate and neocuproine can be distributed in the reagent layer matrix. Uric acid causes reduction of cupric copper to cuprous copper that can complex with the neocuproine to form a colored material that is proportional in density to the concentration of uric acid in the analyzed liquid. In the case of many analyses, enzymes such as oxidase materials like glucose oxidase can desirably be included as interactive materials within a reagent layer of an element intended for the analysis of an analyte that is a substrate for such enzyme. As an example, an oxidative enzyme can be incorporated into a reagent layer together with peroxidase or a peroxidative material and a chromogen material or composition that, upon oxidation in the presence of peroxidase (or another substance having peroxidative activity) and the hydrogen peroxide formed upon interaction of an oxidase and its substrate, provides a dye or other detectable species. An interactive material that, upon appropriate interaction, provides directly a detectable change in the element is also termed an indicator. A plurality of materials, including at least one interactive material, that act together to provide a detectable change in the element is collectively termed an indicator composition.

Chromogenic materials or compositions that contain an oxidizable moiety and can provide a detectable species include certain dye providing materials or compositions. In one aspect, a dye can be provided by a compound, that when oxidized, can couple with itself or with its reduced form to provide a dye. Such autocoupling compounds include a variety of hydroxylated compounds such as orthoaminophenols, alkoxynaphthols, 4-amino-5-pyrazolones, cresols, pyrogallol, guaiacol, orcinol, catechol phloroglucinol, p,p-dihydroxydiphenyl, gallic acid, pyrocatechoic acid, salicyclic acid, etc. Compounds of this type are well known and described in the literature, such as in _The Theory of the Photographic Process_, Mees and James Ed. (1966), especially at Chapter 17. In another aspect, the detectable species can be provided by oxidation of a leuco dye to provide the corresponding dyestuff form. Representative leuco dyes include such compounds as leucomalachite green and leucophenolphthalein. In yet another aspect, the detectable species can be provided by dye providing compositions that include an oxidizable compound capable of undergoing oxidative condensation with couplers such as those containing phenolic groups or activated methylene groups, together with such a coupler. Representative of such oxidizable compounds include benzidene and its homologs, p-phenylenediamines, p-aminophenols, 4-aminoantipyrine, and the like. A wide range of such couplers, including a number of autocoupling compounds, is described in the literature.

Alternatively, some materials or compositions contain a reducible moiety that can provide a radiometrically detectable compound. This compound may be either formed or destroyed by the reductive process. Examples of the former type of chemistry may be found in the direct radiometric measurement, usually at a wavelength of 340 nanometers, of reduced nicotinamide adenine dinucleotide (reduced NAD) as may be formed by the reaction of glucose with glucose dehydrogenase and NAD, as well as in the further reaction of reduced NAD with diaphorase and any one of a variety of tetrazolium compounds and subsequent radiometric detection of the resulting formazan. A specific example of such a tetrazolium is iodonitrotetrazolium chloride (INT) which, upon reduction, produces a red colored formazan. 2,6-Dichlorophenolindophenol is an example of a compound whose color is destroyed upon reduction.

Additional layers can also be arranged to provide for a variety of chemistries or function and to provide a function in its own layer or in combination with another reagent layer. Thus, a plurality of layers can be utilized. Filtering, registration or mordanting functions can be provided for by additional layers. Prior art is replete with examples of multiple layers such as is found in U.S. Patents 4,042,335 and 4,050,898, for example.

As used herein, the term "reagent" and expression "reagent layer" mean a material that is capable of interaction with an analyte, a precursor of an analyte, a decomposition product of an analyte or an intermediate. For example, one of the reagents can be a radiometrically detectable species which is mobilized by the analyte from a radiometrically opaque portion or layer of the element to a radiometrically transparent portion or layer such as a registration layer.

Interaction between the reagents of the reagent composition and the analyte is therefore meant to refer to chemical reaction, catalytic activity as in the formation of an enzyme substrate complex or any other form of a chemical or physical interaction including physical displacement that can produce ultimately a radiometrically detectable signal in the element.

Further modifications and variations of the invention will be apparent from the foregoing and are intended to be encompassed by the claims appended hereto.

## Claims

1. A reagent test device for the testing of fluid samples comprising oppositely disposed top and bottom surface layers defining therebetween a capillary zone of intended liquid transport of a test liquid, said top and bottom surface layers being spaced apart at a distance no greater than which will maintain a capillary flow of said test liquid therebetween and wherein said capillary zone comprises a sample test chamber, and said top surface layer contains interactive material capable of reaction with a component of said test liquid to provide a detectable response, said bottom layer extending beyond said top surface layer to form a handle for said device,

an air vent means extending from said sample test chamber between said top and bottom surface layer and which functions to permit any entrapped air from the test liquid to escape from the sample test chamber,

a sample entry port for introduction of said test liquid into said capillary zone, said sample application port being in communication with said sample test chamber.

2. The device as set forth in claim 1, wherein the distance between said top and bottom surface layers is between about 0.007 and about 0.08 centimeter.

3. The device according to any of claims 1 and 2, further comprising an adhesive layer interposed between said surface layers for sealing said surface layers together.

4. The device according to any of claims 1 to 3, wherein said air vent means and said sample test chamber are formed in a surface layer.

5. The device according to any of claims 1 to 4, wherein the interactive material is a multiple reagent layer.

6. The device according to any of claims 1 to 5, wherein said sample entry port comprises a sample entry port located at the end of said device, said port being a relatively narrow passageway connected to a sample test chamber of relatively larger area, said air vent means being connected to said chamber by a relatively narrow passageway.

7. A reagent test strip device for testing a fluid sample comprising a top reagent surface layer, a bottom strip layer and a spacer layer positioned therebetween and defining a capillary zone of intended liquid transport of a dimension no greater than that which will maintain a capillary flow of liquid introduced into said zone, and wherein said capillary zone comprises a sample test chamber containing interactive material capable of reacting with a component of said liquid to provide a detectable response,

an air vent located in contact with said sample test chamber and between said layers which functions to permit any air from the sample test chamber to escape,

and a sample entry port for introduction of liquid into said capillary zone, said sample entry port being in communication with said sample test chamber.

8. The device as set forth in claim 7, further comprising an adhesive layer being formed on the underside of the top surface layer adapted to be in sealing relation to the spacer layer.

9. The device according to any of claims 7 and 8, wherein the interactive material is a multiple reagent layer.

10. The device according to any of claims 7 to 9, wherein the distance between the top layer and the bottom layer is between about 0.007 and about 0.08 centimeter.

**FIG. I**

**FIG. 2**

**FIG. 3**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | GB-A-2 090 659 (T.F. KELLEY et al.) <br> * Figures 8-10; page 1, lines 1-6,25-31; page 3, lines 32-71,84-91,92-103 * . | 1-10 | B 01 L 3/00 <br> G 01 N 33/52 |
| X | WO-A-8 504 255 (A. BLAKE et al.) <br> * Figure 1; page 7, lines 14-22; page 12, line 31 - page 13, line 19; page 13, line 34 - page 14, line 5 * | 1,7 | |
| X | FR-A-2 325 920 (J.E. LILJA et al.) <br> * Figures 7,8; page 1, lines 21-26; page 1, line 39 - page 2, line 4; page 3, lines 29-33; page 4, line 38 - page 5, line 22 * | 7 | |
| A | EP-A-0 215 419 (J.M. BLATT) | | |

TECHNICAL FIELDS
SEARCHED (Int. Cl.4)

B 01 L
G 01 N

The present search report has been drawn up for all claims

| Place of search | Date of completion of the search | Examiner |
|---|---|---|
| THE HAGUE | 19-07-1988 | HOCQUET A.P.E. |

EPO FORM 1503 03.82 (P0401)